# EUROPEAN PATENT APPLICATION

(11) **EP 3 449 905 A1**
(43) Date of publication of application: **06.03.2019**
(21) Application number: 18198909.6
(22) Date of filing: 21.06.2013
(51) Int. Cl.: A61K 8/87, A61Q 3/02, A61Q 3/00, A61K 8/35, A61K 8/81, A61K 8/55

(54) **RADIATION-CURABLE, STABLE NAIL GEL COMPOSITIONS AND METHODS OF PREPARATION AND USE**

(30) Priority: 22.06.2012 US 201261662988 P; 05.09.2012 US 201261696931 P; 01.04.2013 US 201361807075 P
(62) Divisional of application: 13807308.5
(71) Applicant: Mycone Dental Supply Company Inc., Cherry Hill, NJ 08002 (US)
(72) Inventor: LEIN, George, ELKTON, 21921 (US)
(74) Representative: Lavoix

(57) **Abstract**

A radiation-curable gel nail coating composition comprising
(d) one or more of the following:
i. a vinyl functional, urethane;
ii. a vinyl containing polyester; and
iii. a compound containing an ester which does not contain a hydroxyl group;

(e) at least one hydroxyl-containing monomer, oligomer, or solvent selected from the group consisting of 2-hydroxyethyl (meth)acrylate (HEMA), diethylene glycol mono(meth)acrylate, glycerol (meth)acrylate, glycerol di(meth)acrylate, sorbitol (meth)acrylate, di(meth)acrylate and tri(meth)acrylate, 2-hydroxypropyl (meth)acrylate (HPMA), 3-hydroxypropyl (meth)acrylate, tetraethylene glycol mono(meth)acrylate, pentaethylene glycol mono(meth)acrylate, dipropylene glycol mono(meth)acrylate, dipentaerythritol penta (meth)acrylate, pentaerythritol tri(meth)acrylate, caprolactone (meth)acrylates, polycaprolactone (meth)acrylates, polyethyleneoxide mono(meth)acrylates, polypropyleneoxide (meth)acrylates, ditrimethyol propane tetra(meth)acrylate, carbohydrate based (meth)acrylic monomers, hydroxyl alkyl (meth)acrylamide, and n-methylol acrylamide; and
(f) a photo initiator;
the radiation-curable gel nail coating composition comprising less than 10 ppm tin, preferably tin-free; and a method comprising applying the composition to a finger or toe nail and curing it in the presence of UV radiation.

## Description

### CROSS-REFERENCE TO RELATED APPLICATIONS

Benefit of U.S. provisional applications serial number 61/662,988, filed June 22, 2012, 61/696,931, filed September 5, 2012, and 61/807,075, filed April 1, 2013, are claimed.

### BACKGROUND OF THE INVENTION

This invention relates to the field of radiation-curable gels useful for cosmetic adornment of natural fingernails and toenails, artificial fingernails and toenails, and artificial nail extensions.

Such radiation-curable gels are often comprised of a hydroxyl-containing monomer and a vinyl functional urethane prepared by reacting a hydroxyl compound such as a polyester, polyether, and/or hydroxyl-containing unsaturated monomer with an isocyanate in the presence of a tin catalyst such as dibutyltin dilaurate (DBTL). Reaction products of polyhydric polyesters, polyethers, diisocyanates, and hydroxyl-containing acrylic or methacrylic monomers represent the most commonly used polyester urethanes. Polyether-based polyurethanes can be prepared by reacting polyhydric polyethers, diisocyanates, and hydroxyl-containing acrylic or methacrylic monomers. In addition to urethanes, vinyl polyesters and polyethers can also be produced by the direct reaction of hydroxyl-containing polyethers, polyols, polyacids or polyesters, and acid- or ester-containing monomers. This reaction is often catalyzed by DBTL as well. The radiation-curable gels are either colorless or pigmented and are usually applied by professional nail technicians and cured by holding the hands or toes under actinic radiation. Such radiation-curable gels can be applied directly to natural fingernails or toenails, or alternatively can be applied to nail extensions bonded to fingernails. In some cases, the artificial nails are coated with conventional nail polish after they are cured.

It has been discovered that such radiation-curable gel compositions are unstable over time. Instability of such compositions results in a reduction in viscosity, which is undesirable. In addition, decomposition of ester containing monomers and other ester containing components is also observed.

It is an object of the present invention to improve the stability of radiation-curable nail gel compositions.

It is another object to improve the stability of radiation curable nail gel compositions comprising ethylenically unsaturated hydroxyl-functional monomers or oligomers and vinyl functional polyester, polyethers, and/or vinyl functional urethanes.

### SUMMARY OF THE INVENTION

These objects, and others which will become apparent from the following disclosure, are achieved by the present invention which comprises in one aspect a radiation-curable gel composition comprising a radiation-curable gel nail coating composition comprising (a) one or more of the following: a vinyl functional urethane; a vinyl containing polyester; and/or a compound containing ester functionality which does not contain a hydroxyl group; (b) at least one hydroxyl-containing monomer, oligomer, or solvent; and (c) a photo initiator; the radiation-curable gel nail coating composition comprising less than 10 ppm tin, preferably less than 5 ppm tin, and most preferably comprising less than 1 ppm tin. The components of the composition are preferably prepared either with no catalyst or in the presence of a catalyst other than tin.

In another aspect of the invention the radiation curable gel composition comprises a vinyl containing ester prepared by reaction of an alcohol, a polyol, a polyacid, an anhydride, polyether, or polyester, and acid-, anhydride-, or ester-containing monomers such as an acrylic or methacrylic monomer, the vinyl containing ester having been prepared in the absence of tin or, in some cases, in the presence of tin followed by a tin removal or reduction step.

The radiation curable nail gel containing compositions in some embodiments comprise 5-80 wt. % of the urethanes and/or esters, 2-80 wt. % of the hydroxyl-containing monomer, oligomer, or solvent, and 0.1 to 10 wt. % photo initiator. The balance to make 100% by weight can be from other components.

In another aspect of the invention mixtures of oligomers prepared in the absence of tin are used. These oligomers may include polyether urethanes and/or polyester urethanes.

In some embodiments polyether urethanes prepared in the absence of tin can be used in the absence of polyester urethanes.

In some embodiments, aliphatic or aromatic urethanes may be used.

In some embodiments urethanes are prepared, in the absence of tin, from the reaction of isocyanate-terminated prepolymers containing polyesters and/or polyethers or from isocyanates which do not contain polyester or polyether groups with hydroxyl-containing (meth)acrylate monomers.

It has been discovered that tin is at least in part responsible for instability and resultant loss of viscosity of such compositions over time and therefore minimization or elimination of the conventional tin catalyst in radiation curable nail coating gel compositions is critical.

While the tin catalysts are especially detrimental to the stability of the polyester based polyurethanes, in the radiation curable gel compositions comprising polyether based polyurethanes or aliphatic or aromatic urethanes the tin causes unwanted decomposition of the hydroxyl-containing monomers, other monomers, or additives which contain ester functionality.

In radiation curable nail gel composition embodiments comprising a hydroxy-containing monomer, the monomer and the urethane are each ethylenically unsaturated since the compositions of such embodiments are cured by copolymerization through the ethylenic unsaturation of the monomer and oligomer. The ethylenic unsaturation of the urethane is preferably provided by a vinyl group of a (meth)acrylate unit. The ethylenic unsaturation of the hydroxy functional monomer or oligomer is preferably provided by (meth)acrylate unit, for example hydroxy alkyl (meth)acrylates are suitable.

The vinyl functional urethane is preferably prepared by reaction of the hydroxyl-containing polyester, polyether and/or hydroxyl-containing (meth)acrylate monomer and the isocyanate in the presence of a catalyst selected from the salts or complexes of the group consisting of bismuth, zinc, hafnium, zirconium, copper, iron, chromium, aluminum, cerium, titanium, manganese, nickel, potassium, and cobalt. Amine catalysts may also be used. Examples of such catalysts include metal based catalysts such as carboxylate complexes and salts of Bi, Zn, Ce, Co, K, and Pb including complexes with 2-ethylhexanoic acid, neodecanoic acid, acetic acid, napthanoic acid, n-octanoic acid, butyric acid, pivalic acid, and other carboxylic acids, dione complexes of Zr, Ti, Zn, Mn, Ni, Fe, Cu, and Cr, including complexes with 2,4-pentanedione, 6-methyl-2,4-heptadione, 2,2,6,6,-tetramethyl-3,5,-heptanedione, 1-benzoyl acetone, ethyl acetoacetate, 3-ethyl-2,4-pentanedione, 1,1,1-trifluoro-2,4,-pentanedione, triacetyl methane and other beta carbonyl diketones. Amine based catalysts, when used, may be selected from any tertiary amine, for example 1,4-diazabicyclo[2.2.2]octane. Other examples of amine based catalysts include Bis-(2-dimethylaminoethyl)ether, benzyldimethylamine, N,N-dimethylcyclohexylamine, pentamethyldiethylenetriamine, N,N,N'-trimethyl-N'-hydroxyethylbisaminoethylether, 1,3-propanediamine, N'-(3-(dimethylamino)propyl)-N,N-dimethyl, N-ethylmorpholine, N-methylmorpholine, 2,2'-dimorpholinodiethylether, 1,3,5-tris(3-(dimethylamino)propyl)-hexahydro-s-triazine available from Huntsman Corporation as well as catalysts available from Air Products such as 1,8 diazabicycloundec-7-ene and others sold under the DABCO® trademark. Mixtures of catalysts may also be used.

The urethanes may contain one or more ester groups.

Bismuth, zinc, and amine-based catalysts are preferred with bismuth based catalysts being most preferred.

In some embodiments the vinyl functional urethane is prepared by reaction of a hydroxyl-containing monomer, polyester, and/or polyether with an isocyanate in the absence of catalyst.

Hydroxyl containing polyesters useful in the invention may be prepared by conventional methods known in the art such as condensation polymerization, ring opening polymerization, reaction with anhydrides and other methods. Preferably the hydroxyl containing polyesters are prepared in the absence of a tin catalyst.

Vinyl containing polyesters can be prepared from the reaction of a (A) diol, polyol, or polyether polyol with (B) a diacid-, anhydride-, polyacid-, and/or an acid-, anhydride-, or ester-containing monomer with vinyl groups, with the ratio of (A) to (B) adjusted to give the desired molecular weight. Other vinyl containing polyesters can be prepared from the ring opening polymerization of, for example, caprolactone. Preferably the vinyl containing polyesters are prepared in the absence of any tin catalyst.

Examples of polyether polyols include any hydroxyl-containing polyether. In some embodiments the polyethers are made via opening of epoxides.

Vinyl containing esters containing polyethers may be prepared via known methods including reaction of hydroxyl-containing polyethers with acid-, anhydride-, or ester containing-monomers, vinyl containing anhydrides and other methods known in the art. The vinyl containing esters containing polyethers may contain one or more ester groups.

Examples of other hydroxyl-containing monomers, oligomers, and solvents include polyacrylates made by incorporating hydroxyl-containing monomers in the polymer. It has been discovered that a hydroxyl-containing monomer, oligomer, or solvent causes side reactions when it reacts with an ester and causes decomposition.

Examples of isocyanates useful in the invention include isophorone diisocyanate, hexamethylene diisocyanate, trimethyl hexamethylene diisocyanate, 4,4'-methylene dicyclohexyl diisocyanate, toluene diisocyanate, methylene diphenyl diisocyanate, polymeric methylene diphenyl diisocyanate, tetramethylxylylene diisocyanate, triisocyanurate, isocyanatoethyl methacrylate, isophorone diisocyanate trimer, hexamethylenediisocyanate trimer, hexamethylene diisocyanate biuret, and hexamethylene diisocyanate uretdione. Isocyanate terminated prepolymers prepared from polyester, polyether or other hydroxyl functional materials may also be used. Mixtures of materials containing isocyanate groups may also be used,

In embodiments of the radiation curable gel composition adapted for coating natural or artificial fingernails, it is preferred that the gel have an initial viscosity of 5 to 2500 poise. In embodiments adapted for use as a builder gel, the initial viscosity is preferably about 250 to 2500 poise. In embodiments adapted for use as a topcoat nail gel the initial viscosity is preferably about 5-25 poise. In embodiments of the composition adapted for use as a pigment containing polish gel the initial viscosity is preferably about 10-40 poise. In general these viscosity differences are achieved by varying the ratio of the polyurethane or oligomers and the hydroxyl-containing monomer and other monomers. Alternatively these viscosity differences can be achieved by the use of thixotropic additives.

The improved stability of the gel compositions results in their maintaining at least 85% of their initial viscosity over one year of storage, and more preferred compositions do not lose more than 10% of their initial viscosity over a year. Most preferred compositions lose no more than 5% of their initial viscosity over one year. In cases where thixotropic additives are used to adjust the initial viscosity, the loss in viscosity on storage is observed at higher shear rates corresponding to shear rates encountered at application conditions. Thus maintaining stability of viscosity over the entire shear regime of 2 sec⁻¹ to at least 70 sec⁻¹ on storage is required.

The improved stability of the gel compositions also results in lower levels of decomposition of the ester containing monomers and lower levels of formation of byproducts such as crosslinkers. For example, decomposition of hydroxyethyl methacrylate results in the formation of the unwanted by-product ethylene glycol dimethacrylate which can negatively affect the balance of properties of the final cured material. It is preferable that less than 10% of the ester containing monomer is decomposed over one year, more preferably less than 5% and most preferably less than 1%.

Examples of the hydroxyl-containing monomers include 2-hydroxyethyl acrylate, 2-hydroxyethyl methacrylate, diethylene glycol monoacrylate, diethylene glycol monomethacrylate, glycerol (meth)acrylate, glycerol di(meth)acrylate, sorbitol (meth)acrylate, di(meth)acrylate and tri(meth)acrylate, 2-hydroxypropyl acrylate, 2-hydroxypropyl methacrylate, 3-hydroxypropyl acrylate, 3-hydroxypropyl methacrylate, tetraethylene glycol mono(meth)acrylate, pentaethylene glycol mono(meth)acrylate, dipropylene glycol monomethacrylate, and dipropylene glycol monoacrylate, dipentaerythritol penta acrylate, dipentaerythritol penta methacrylate, pentaerythritol triacrylate, pentaerytritol trimethacrylate, caprolactone (meth)acrylates, polycaprolactone (meth)acrylates, polyethyleneoxide mono(meth)acrylates, polypropyleneoxide (meth)acrylates, ditrimethyol propane tetra(meth)acrylate, carbohydrate based (meth)acrylic monomers, and hydroxyl alkyl (meth)acrylamides such as n-methylol acrylamide. The most preferred hydroxyl-containing monomers are hydroxyethyl methacrylate (HEMA) and hydroxypropyl methacrylate (HPMA). Mixtures of more than one hydroxyl-containing monomer can be used. It is preferable that the hydroxyl functional monomers be present as greater than 5% of the formulation.

Hydroxy functional polyurethane, polyester, and/or polyether oligomers may also be used.

The compositions can include a hydroxyl containing solvent, an ester containing solvent, and/or a solvent containing neither hydroxyl nor ester. Typical such solvents are butyl acetate and ethyl acetate. Examples of other such solvents are isopropanol, toluene, methyl ethyl ketone, acetone, xylene, propyl acetate, butanol, and diacetone alcohol, propylene glycol, and butyl carbitol.

The composition can also include typical ingredients found in commercial nail polish such as cellulose and its derivatives, for example cellulose acetate butyrate, cellulose acetate propionate, and nitrocellulose, and/or polyester resins, polymers containing acid groups, aliphatic solvents, aromatic solvents, commercial nail polish, nail polish concentrates, and the like.

The compositions can be applied to a human finger nail or toe nail as a coating or can be applied to artificial nails. The radiation curing step can be conducted using any conventional ultraviolet (UV) cure apparatus and cure conditions known for use in the nail coating industry. The use of non-conventional catalysts, i.e., catalysts other than tin, to prepare the vinyl functional urethane does not significantly affect the UV cure rate or conditions.

### DETAILED DESCRIPTION

The present invention comprises in one aspect a radiation-curable gel composition comprising a radiation-curable gel nail coating composition comprising (a) one or more of the following: a vinyl functional urethane; a vinyl containing polyester; and/or a compound containing ester functionality which does not contain a hydroxyl group; (b) at least one hydroxyl-containing monomer, oligomer, or solvent; and (c) a photo initiator; the radiation-curable gel nail coating composition comprising less than 10 ppm tin, preferably less than 5 ppm tin, and most preferably comprising less than 1 ppm tin. The components of the composition are preferably prepared either with no catalyst or in the presence of a catalyst other than tin.

It has been discovered that tin is at least in part responsible for instability and resultant loss of viscosity of radiation curable nail coating gel compositions over time. Such compositions are often stored for several months before they are used. Therefore the tin free and low tin gel compositions have improved retention of viscosity and improved stability versus conventional tin catalyzed oligomers.

In some embodiments the vinyl functional urethanes can have at least one, preferably two or more acryloyl, or methacryloyl groups and a urethane group. Examples include urethanes based on aliphatic, aromatic, polyester, and polyether polyols and aliphatic, aromatic, polyester, and polyether diisocyanates capped with (meth)acrylate end groups and urethane (meth)acrylates made from reaction of aliphatic or aromatic isocyanates with hydroxyl-containing (meth)acrylic monomers or oligomers. Epoxy urethane (meth)acrylates, useful in the present invention, have at least one, preferably two or more two or more acryloyl or methacryloyl groups and a urethane group. Examples include epoxy (meth)acrylates based on aliphatic or aromatic epoxy prepolymers capped with a urethane (meth)acrylate end group. An aliphatic or aromatic urethane spacer can be optionally inserted between the epoxy and the (meth)acrylate end group(s). (Meth)acrylated polyester oligomers, useful in the present invention have at least one, preferably two or more acryloyl or methacryloyl groups and a polyester core. (Meth)acrylated acrylate oligomers, useful in the present invention, have at least two or more acryloyl or methacryloyl groups and a polyacrylic core. These materials can be made by methods well known in the art. Compounds containing hydroxyl groups, including polyesters, polyethers, epoxies, aliphatic or aromatic compounds can be reacted with a diisocyanate to form an isocyanate prepolymer which is subsequently reacted with a hydroxyl-containing (meth)acrylate. Alternatively, a (meth)acrylic monomer containing an isocyanate group can be reacted with the compounds containing hydroxyl groups. Other vinyl containing isocyanates such as 3-Isopropenyl-alpha, alpha dimethybenzyl isocyanate may also be used. The hydroxyl-containing (meth)acrylic monomers can also be reacted with isocyanates to form the urethane. Examples of this latter group include urethane dimethacrylate, bis hydroxyethyl methacrylate isophorone diurethane (bis-HEMA IPDI) and the reaction product of isophorone diisocyanate and hydroxethyl acrylate.

Mixtures of these urethanes and mixtures of these urethanes with (meth)acrylated polyesters may also be used.

Suitable compounds containing ester functionality which do not contain a hydroxyl group comprise, for example, solvents such as butyl acetate, ethyl acetate, isopropyl acetate, iso butyl acetate, monomers such as ethyl (meth)acrylate, methyl (meth)acrylate, butyl (meth)acrylate, butoxyethyl (meth)acrylate, 2-ethylhexyl (meth)acrylate, methoxyethylene glycol (meth)acrylate, methoxy propyl (meth)acrylate, phenoxyethylene glycol (meth)acrylate, stearyl (meth)acrylate, isobornyl (meth)acrylate. tetrahydrofufuryl (meth)acrylate , ethylene glycol di(meth)acrylate, 1,4 butane diol di(meth)acrylate, 1,6 hexananediol di(meth)acrylate, 1,9 nonanediol di(meth)acrylate, 1,10 decanediol di(meth)acrylate, neopentyl glycol di(meth)acrylate, 2-methyl-1,8-octane diol di(meth)acrylate, propylene glycol di(meth)acrylate, and oligomers and polymers made from (meth)acrylic monomers which do not contain hydroxyl groups.

Use of any of these materials requires that their use levels do not result in an increase in the level of tin in the composition such that instability of the resulting composition occurs. Preferably these materials are prepared in the absence of tin catalysts.

In addition to the above-described (meth)acrylate- based polymerizable materials, other polymerizable monomers, oligomers or polymers of monomers which contain at least one free radical polymerizable group in the molecule may be used without any limitations in the curable gel provided that their use does not increase the level of tin in the composition to greater than 10 ppm, preferably no greater than 5 ppm and most preferably no greater than 1 ppm. Typical examples include esters of acrylic and methacrylic acid, herein termed (meth)acrylic ester. Specific but not limiting examples of mono (meth)acryloyl esters include methyl (meth)acrylate, ethyl (meth)acrylate, hydroxypropyl (meth)acrylate, butyl (meth)acrylates, hydroxy ethyl (meth)acrylates, butoxyethyl (meth)acrylate, diethylaminoethyl (meth)acrylate, 2-ethylhexyl (meth)acrylate, ethoxyethyl (meth)acrylate, t-butyl aminoethyl (meth)acrylate, methoxyethylene glycol (meth)acrylate, phosphoethyl (meth)acrylate, methoxy propyl (meth)acrylate, methoxy polyethylene glycol(meth)acrylate, phenoxyethylene glycol (meth)acrylate, phenoxypolyethylene glycol (meth)acrylate, 2-hydroxy-3-phenoxypropyl (meth)acrylate, 2-(meth)acryloxyethylsuccinic acid, 2-(meth)acryloylethylphthalic acid, 2-(meth)acryloyloxypropylphthalic acid, stearyl (meth)acrylate, isobornyl (meth)acrylate, 3-chloro-2-hydroxypropyl (meth)acrylates, tetrahydrofufuryl (meth)acrylate, (meth)acrylamides and allyl monomers. Specific but not limiting examples of difunctional (meth)acryloyl esters include 1,4 butane diol di(meth)acrylate, 1,6 hexananediol di(meth)acrylate, 1,9 nonanediol di(meth)acrylate, 1,10 decanediol di(meth)acrylate, neopentyl glycol di(meth)acrylate, 2-methyl-1,8-octane diol di(meth)acrylate, glycerin di(meth)acrylate, ethylene glycol di(meth)acrylate, triethyleneglycol di(meth)acrylate, polyethylene glycol di(meth)acrylate, propylene glycol di(meth)acrylate, polypropylene glycol di(meth)acrylate, ethoxylated propylene glycol di(meth)acrylate, ethoxylated polypropylene glycol di(meth)acrylate, polyethoxypropoxy di(meth)acrylate, ethoxylated bisphenol A di(meth)acrylate, propoxylated bisphenol A di(meth)acrylate, propoxylated ethoxylated bisphenol A di(meth)acrylate, bisphenol A glycidyl methacrylate, tricyclodecanedimethanol di(meth)acrylate, glycerin di(meth)acrylate, ethoxylated glycerin di(meth)acrylate, bis acrylamides, bis allyl ethers and allyl (meth)acrylates. Examples of tri and or higher (meth)acryloyl esters include trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tri(meth)acrylate, pentaerythritol tetra(meth)acrylate, propoxylated pentaerythritol tetra(meth)acrylate, ethoxylated pentaerythritol tetra(meth)acrylate, dipentaerythritol hexa(meth)acrylate, and ethoxylated isocyanuric acid tri(meth)acrylates. These monomers may contain an acidic group to improve adhesion. Examples of these include Sarbox® monomers available from Sartomer Company.

A compound having at least one free radical polymerizable group includes not only a single component but also a mixture of polymerizable monomers. Thus combinations of two or more materials containing free radical polymerizable groups may be used.

The gels also contain a photo initiator. Examples of these include benzyl ketones, monomeric hydroxyl ketones, polymeric hydroxyl ketones, alpha-amino ketones, acyl phosphine oxides, phosphinates, metallocenes, benzophenone, benzophenone derivatives, and the like. Specific examples include 1-hydroxy-cyclohexylphenylketone, benzophenone, 2-benzyl-2-(dimethylamino)-1-(4-(4-morphorlinyl)phenyl)-1-butanone, 2-methyl-1-(4-methylthio)phenyl-2-(4-morphorlinyl)-1-propanone, diphenyl-(2,4,6-trimethylbenzoyl) phosphine oxide, phenyl bis(2,4,6-trimethylbenzoyl) phosphine oxide, benzyl-dimethylketal, isopropylthioxanthone, ethyl (2,4,6-trimethyl benzoyl) phenyl phosphinate and phenyl (2,4,6-trimethyl benzoyl) phenyl phosphinate and mixtures thereof.

Photo accelerators such as aliphatic or aromatic amines may also be included in the gel as well as fillers, inhibitors, plasticizers and adhesion promoters. Some of such components may contain ester or hydroxyl groups.

By the term "gel," we mean a radiation-curable composition comprising photo initiator, ethylenically unsaturated monomers and/or oligomers, having a viscosity suitable for coating natural or artificial nails, or forming artificial nails and extensions, as well as adorning such nails.

Pigments and dyes may be used to color the gels. These may be added directly to the formulation.

Pigment concentrates can be used to provide color to the composition and when used generally contain 10-50% pigment which may be dispersed in an organic liquid comprised of one or more chemicals selected from solvents, ethylenically unsaturated monomers, and ethylenically unsaturated oligomers. The organic liquid may also comprise non-reactive polymer, filler, and dispersant. For example, the organic liquid may comprise nitrocellulose. The organic liquid has one continuous phase whereas the pigment is a discontinuous phase of the pigment concentrate. Examples of suitable solvents are ethyl acetate and butyl acetate. Examples of ethylenically unsaturated monomers are (meth)acrylic esters, and examples of ethylenically unsaturated oligomers are urethane (meth)acrylates. The concentrates may be dispersed in the same UV-curable monomers and/or oligomers as used in the gel formulation by any means, for example by shearing of the pigment directly into the organic liquid. In one embodiment the organic liquid comprises ethyl acetate, butyl acetate, and nitrocellulose.

Suitable pigments which can be incorporated into the concentrates include barium, calcium and aluminum lakes, iron oxides, chromates, molybdates, cadmiums, metallic or mixed metallic oxides, talcs, carmine, titanium dioxide, chromium hydroxides, ferric ferrocyanide, ultramarines, titanium dioxide coated mica platelets, and/or bismuth oxychlorides. Preferred pigments include D&C Black No. 2, D&C Black No. 3., FD&C Blue No. 1, D&C Blue No. 4, D&C Brown No. 1, FD&C Green No. 3, D&C Green No. 5, D&C Green No. 6, D&C Green No. 8, D&C Orange No. 4, D&C Orange No. 5, D&C Orange No. 10, D&C Orange No. 11, FD&C Red No. 4., D&C Red No. 6, D&C Red No. 7, D&C Red No. 17, D&C Red No. 21, D&C Red No. 22, D&C Red No. 27, D&C Red No. 28, D&C Red No. 30. D&C Red No. 31, D&C Red No. 33, D&C Red No. 34, D&C Red No. 36, FD&C Red No. 40, D&C Violet No. 2, Ext. D&C Violet No. 2, FD&C Yellow No. 5, FD&C Yellow No. 6, D&C Yellow No. 7, Ext. D&C Yellow No. 7, D&C Yellow No. 8, D&C Yellow No. 10, D&C Yellow No. 11, as well as others listed on the FDA color additives website, and Annex IV of the Cosmetic Directive 76/768/EEC, Coloring Agents Permitted in Cosmetics.

Thixotropic additives may also be used in the compositions. A thixotropic additive is defined herein as an additive that when mixed with a relatively low viscosity gel imparts shelf stability to the pigmented gel. The pigment does not readily fall out of the gel to form a hard pack. The thixotropic additive also imparts shear thinning properties such that a viscosity reduction of at least a factor of 2 occurs over the range of shear from 1/2sec to 1/70sec, i.e.- the gel is thick (viscous) under normal storage conditions, but flows (becomes thin, less viscous) when stressed such as applying the gel to nails. The thixotropic additive changes the rheological properties of the gel.

Thixotropic additives useful in this invention include inorganic and organic materials. Examples of inorganic materials useful in the invention include but are not limited to calcium, zinc or aluminum stearate, silica, fumed silica such as that available as Aerosil ® from Evonik Industries or Cab-O-Sil® available from Cabot Corporation, diatomaceous earth, bentonite clay, kaolinite, pyrophyllite, sericite, saponite, smectic/vermiculites (montmorillinite, beidillite, nontronite, hectorite and saponite), organic modified bentonite and hectorite such as stearyl alkonium hectorite and others that are available from Elementis Specialties under the trade name of Bentone®, talc, mica, zirconium oxide, zinc oxide, and magnesium oxide. Examples of organic materials useful in the invention include but are not limited to hydrogenated castor oils, hydrogenated castor oil waxes, inorganically modified castor oils, organically modified castor oils such as those sold by Elementis Specialties under the Thixcin® trademark, triglycerides such as glyceryl tri-12-hydroxy stearate, polyamides and modified polyamides such as 12-hydroxystearic acid diamide of ethylene diamine, 12-hydroxystearicacid diglycolamide, N-stearyl ricinoleamide, N-stearyl stearamide and other polyamide waxes. Included in these polyamide materials are those sold commercially by Kusumoto Chemicals Industries under the Disparlon® trademark, by Lehmann and Voss under the Luvotix® trademark, by Elementis Specialties under the Thixatrol® trademark, polyethylene oxide waxes, urea urethanes believed to be exemplified by those sold by Byk Incorporated as, for example, by Byk-410, Byk-411, and Byk-420, acrylic resins, amine salts of polymeric polyesters, salts of linear polyaminoamide and polymeric polyester, amide solutions of polycarboxylic acid, alkyl sulfonate, alkylallyl sulfonate, colloidal ester, polyester resin such as those sold by Elementis Specialties under the Thixatrol® trademark, phenol resin, melamine resin, epoxy resin, urethane resin, styrene butadiene polymers, polyimide resin, and polyester amides. Materials such as those sold by Byk under the trademarks of Anti-Terra® and Bykumen® can also be used.

Thixotropic additives can be used at amounts from 0.1 to 10 wt.%. It is preferred to use quantities from 0.5 to 5.0 wt.% and more preferred to use amounts of 0.5 to 3.0 wt %. The preferred thixotropic additives are polyamides, urea urethanes, and silica, or a mixture thereof..

### EXAMPLES

The following non-limiting examples in which all parts and percentages are by weight unless otherwise indicated are presented to illustrate certain embodiments of the invention and comparative examples (indicated by a "*") representing the prior art.

### Example 1 - Preparation of Polyester oligomer with dibutyltin dilaurate (DBTDL) (Comparative *)

To a resin kettle equipped with a stirrer was charged, under dry air, 0.3 moles of isophorone diisocyanate (IPDI) and 0.160 g of DBDTL and 0.8g of butylated hydoxy toluene (BHT). The mixture was heated to 50°C with stirring and 0.3 moles of hydroxyethyl acrylate (HEA) was added over 1 hr. After addition, 0.15 moles of a 1000 Mw polyethylene adipate diol which had been prepared without catalyst (Fomrez 22-114U, Chemtura Co.) was added. The reaction was held at 65°C until no isocyanate peak remained in the infrared spectrum.

### Example 2 - Preparation of Polyester oligomer with Bismuth Neodecanoate

The procedure of Example 1 was used substituting 0.34g of Bicat 8108 (Shepherd Chemical) for the DBTDL.

### Example 3 - Preparation of Polyester oligomer with Diazobicycloundecene

The procedure of Example 1 was followed substituting 0.16g of diazobicycloundecene for the DBTDL.

### Example 4 - Preparation of Polyester oligomer with bismuth neodecanoate/zinc neodecanoate

The procedure of example 1 was followed substituting 0.158g Bicat 8 (Shepherd Chemical) for the DBTDL.

### Example 5 - Preparation of Polyester oligomer using DBTDL (Comparative *)

To a resin kettle was charged 0.6 moles of IPDI and 0.3 moles of a 1000 Mw polybutylene adipate diol prepared without catalyst (Fomrez 44-114U, Chemtura Co.) under dry air. The reaction was heated to 50°C and 0.32g of DBTDL was added. After the exotherm the reaction was allowed to cool to 60°C and 0.8g BHT was added followed by addition of 0.6 moles of HEA over 35 minutes. The reaction was held at 85°C until no isocyanate peak remained in the infrared spectrum.

### Example 6 - Preparation of Polyester Oligomer Using Bismuth neodecanoate

The procedure of Example 5 was repeated substituting 0.5g of Bicat 8108 (Shepherd Chemical) for the DBTDL.

### Example 7 - Preparation of Polyester Oligomer using Diazobicycloundecene

To a resin kettle was charged 0.4 moles of IPDI and 0.2 moles of a 1000 Mw polybutylene adipate diol prepared without catalyst (Fomrez 44-114U) under dry air. The reaction was heated to 50°C and 0.19g of Diazobicycloundecene was added. After the exotherm the reaction was allowed to cool to 60°C and 0.8g BHT was added followed by addition of 0.4 moles of HEA over 35 minutes. The reaction was held at 70°C until no isocyanate peak remained in the infrared spectrum.

### Example 8 - Preparation of Polyester oligomer with bismuth neodecanoate/zinc neodecanoate

The procedure of Example 7 was repeated substituting 0.48g of Bicat 8 (Shepherd Chemical) for the diazobicycloundecene.

### Examples 9-12 - Stability Studies for Polyester oligomers

Curable nail formulations were prepared as shown in Table 1. All values are in weight percent.

**Table 1 - Formulations used for Examples 9-12**

| | | | | Oligomer From | | | | | | | |
|---|---|---|---|---|---|---|---|---|---|---|---|
| Example | EGDMA¹ | HEMA² | PI³ | Example* 1 | Example 2 | Example 3 | Example 4 | Example* 5 | Example 6 | Example 7 | Example 8. |
| 9* | 5 | 14 | 3 | 39 | | | | 39 | | | |
| 10 | 5 | 14 | 3 | | 39 | | | | 39 | | |
| 11 | 5 | 14 | 3 | | | 39 | | | | 39 | |
| 12 | 5 | 14 | 3 | | | | 39 | | | | 39 |

| | | | | | | | | | | | |
|---|---|---|---|---|---|---|---|---|---|---|---|
| 1. Ethylene Glycol dimethacrylate 2. Hydroxyethyl methacrylate 3. 2-hydroxy-2-methyl-1-phenylpropan-1-one photo initiator (PI) | | | | | | | | | | | |

Viscosities were measured at 25°C at shear rates of 2, 19, 36, 53, 70 sec⁻¹ on a TA Instruments AR1500EX rheometer after initial preparation and after one month ageing at 50° C. One month accelerated ageing at 50° C. is equivalent to six months normal ageing at room temperature. Minimal (<1 unit) change in viscosity is seen over this range of shear and an average of all five values was used. Tin levels were measured using Inductively Coupled Plasma by Robertson Microlit Laboratories. Table 2 shows the results.

**Table 2 - Viscosity Results in poise for Examples 9-12**

| Example | Tin Level | Initial Viscosity | Aged Viscosity | % Change |
|---|---|---|---|---|
| 9* | 87 ppm | 126 | 94 | 25.4 |
| 10 | <1 ppm | 147 | 147 | 0 |
| 11 | <1 ppm | 169 | 162 | 4 |
| 12 | <1 ppm | 157 | 157 | 0 |

As can be seen only the tin containing sample showed instability on ageing.

### Examples 13-15 - Stability Studies for Polyester oligomers

Curable nail formulations were prepared as shown in Table 3. All values are in weight percent.

**Table 3 - Formulations used for Examples 13-15**

| | | | | Oligomer From | | |
|---|---|---|---|---|---|---|
| Example | EGDMA¹ | HEMA² | PI³ | Example 2 | Example 5* | Example 6 |
| 13* | 5 | 14 | 3 | 39 | 39 | |
| 14* | 5 | 14 | 3 | 39 | 19.5 | 19.5 |
| 15 | 5 | 14 | 3 | 39 | | 39 |

| | | | | | | |
|---|---|---|---|---|---|---|
| 1. Ethylene Glycol dimethacrylate 2. Hydroxyethyl methacrylate 3. 2-hydroxy-2-methyl-1-phenylpropan-1-one | | | | | | |

Viscosities were measured at 25°C at shear rates of 2, 19, 36, 53, 70 sec⁻¹ on a TA Instruments AR1500EX rheometer after initial preparation and after two months ageing at 50°C. Two months accelerated ageing at 50° C. is approximately equivalent to one year normal ageing at room temperature. Minimal (<1 unit) change in viscosity is seen over this range of shear and an average of all five values was used. Tin levels were measured using Inductively Coupled Plasma by Robertson Microlit Laboratories. Table 4 shows the results.

**Table 4 - Viscosity Results in poise for Examples 13-15**

| Example | Tin Level | Initial Viscosity | Aged Viscosity | % Change |
|---|---|---|---|---|
| 13* | 48 ppm | 117 | 101 | 13.7 |
| 14* | 23 ppm | 130 | 117 | 10 |
| 15 | <1 ppm | 136 | 133 | 2.2 |

As can be seen even low levels of tin lead to instability of the system.

### Examples 19-22 - Stability study of thixotrope containing polyester oligomer formulations

Curable nail formulations were prepared as shown in Table 5.

**Table 5 - Formulations used for Examples 16-19**

| | | | Formulation From | | | |
|---|---|---|---|---|---|---|
| Example | Silica¹ | Salcare S95² | Example 9* | Example 10 | Example 11 | Example 12 |
| 16* | 4.5 | 5 | 90.5 | | | |
| 17 | 4.5 | 5 | | 90.5 | | |
| 18 | 4.5 | 5 | | | 90.5 | |
| 19 | 4.5 | 5 | | | | 90.5 |

| | | | | | | |
|---|---|---|---|---|---|---|
| 1. Cab-O-Sil M-5, Available from Cabot Corporation, Boston, MA 2. Polyquaternium 37, Available from BASF Corporation, Ludwigschafen, Germany | | | | | | |

Viscosities were measured at 25°C at a shear rate of 70 sec⁻¹ on a TA Instruments AR1500EX rheometer after initial preparation and after one month ageing at 50°C. Tin levels were measured using Inductively Coupled Plasma by Robertson Microlit Laboratories. Table 6 shows the results.

**Table 6 - Viscosity Results in poise for Examples 16-19**

| Example | Tin Level | Initial Viscosity | Aged Viscosity | % Change |
|---|---|---|---|---|
| 16* | 77 ppm | 43 | 36 | 16.3 |
| 17 | <1 ppm | 57 | 56 | 1.8 |
| 18 | <1 ppm | 60 | 59 | 1.7 |
| 19 | <1 ppm | 54 | 53 | 1.9 |

### Example 20 - Preparation of Polyether oligomer with bismuth neodecanoate

To a resin kettle equipped with a stirrer was charged, under dry air, 0.3 moles of isophorone diisocyanate (IPDI) and 0.4 g bismuth neodecanoate (Bicat 8108) and 0.79g of butylated hydoxy toluene (BHT). Then 0.3 moles of hydroxyethyl acrylate (HEA) was added over 30 min. After addition, 0.15 moles of a 650 Mw polybutylene glycol ether diol (Terathane 650, Invista Corporation) was added and the reaction was held at 75°C until no isocyanate peak remained in the infrared spectrum.

### Example 21 - Preparation of Polyether oligomer with DBTDL (Comparative)

The procedure of Example 20 was repeated substituting 0.19g DBTDL for the bismuth neodeanoate.

### Example 22 - Preparation of Polyether oligomer with Bismuth Neodecanoate

To a resin kettle equipped with a stirrer was charged, under dry air, 0.4 moles of isophorone diisocyanate (IPDI) and 0.2 moles of a 2000 Mw polybutylene glycol diol (Terathane 2000, Invista Corporation). The mixture was heated with stirring to 50°C and 0.82 g bismuth neodecanoate. The reaction exothermed to 60°C, 0.83 g of butylated hydroxyl toluene was added and, after cooling to 50°C, 0.4 moles of hydroxyethyl acrylate was added over 1 hr. The reaction was held at 75°C until no isocyanate peak remained in the infrared spectrum.

### Example 23 - Preparation of Polyether Oligomer with DBTDL (Comparative *)

The procedure of Example 22 was repeated substituting 0.36 g of DBTDL for the bismuth neodecanoate.

### Examples 24 - 27 - Stability of monomer studies

Curable formulations were as shown in Table 7. Gas chromatography was used to analyze for HEMA, SR268 and TPO. Initial chromatograms showed no ethylene glycol dimethacrylate in any of the formulations. After ageing for two months at 50°C both samples, Ex. 21 and Ex. 23, containing oligomers made with DBTDL showed the formation of ethylene glycol dimethacrylate along with another material believed to be due to reaction with SR268, while those made with bismuth catalyst, Ex. 20 and Ex. 22, showed neither of these byproducts. Two months accelerated ageing at 50°C. is equivalent to one year normal ageing at room temperature.

**Table 7 - Formulations used for Examples 24-27**

| | | | | Oligomer From | | | |
|---|---|---|---|---|---|---|---|
| Example | SR268¹ | HEMA² | PI³ | Example* 21* | Example* 20 | Example*23* | Example* 22 |
| 24* | 5 | 15 | 3 | 77 | | | |
| 25 | 5 | 15 | 3 | | 77 | | |
| 26* | 5 | 15 | 3 | | | 77 | |
| 27 | 5 | 15 | 3 | | | | 77 |

| | | | | | | | |
|---|---|---|---|---|---|---|---|
| 1. Tetraethylene glycol diacrylate 2. Hydroxyethyl methacrylate 3. Diphenyl (2,4,6-trimethylbenzoyl)-phosphine Oxide | | | | | | | |

## Claims

1. A radiation-curable gel nail coating composition comprising
(a) one or more esters selected from the group consisting of:
i. a vinyl functional urethane;
ii. a vinyl containing polyester; and
iii. a compound containing an ester which does not contain a hydroxyl group;
(b) at least one hydroxyl-containing monomer, oligomer, or solvent selected from the group consisting of 2-hydroxyethyl (meth)acrylate (HEMA), diethylene glycol mono(meth)acrylate, glycerol (meth)acrylate, glycerol di(meth)acrylate, sorbitol (meth)acrylate, di(meth)acrylate and tri(meth)acrylate, 2-hydroxypropyl (meth)acrylate (HPMA), 3-hydroxypropyl (meth)acrylate, tetraethylene glycol mono(meth)acrylate, pentaethylene glycol mono(meth)acrylate, dipropylene glycol mono(meth)acrylate, dipentaerythritol penta (meth)acrylate, pentaerythritol tri(meth)acrylate, caprolactone (meth)acrylates, polycaprolactone (meth)acrylates, polyethyleneoxide mono(meth)acrylates, polypropyleneoxide (meth)acrylates, ditrimethyol propane tetra(meth)acrylate, carbohydrate based (meth)acrylic monomers, hydroxyl alkyl (meth)acrylamide, and n-methylol acrylamide; and
(c) a photo initiator;
the radiation-curable gel nail coating composition comprising less than 10 ppm tin.

2. The composition of claim 1 comprising less than 1 ppm tin.

3. The radiation-curable gel composition of claim 1 wherein the one or more esters is a vinyl functional polyester urethane, a polyether urethane, or a combination of polyester and polyether urethanes.

4. The composition of claim 1 wherein the vinyl functional urethane comprises a (meth)acrylate unit.

5. The composition of claim 1 wherein the vinyl functional urethane is prepared by reacting at least one hydroxyl-containing polyester and/or polyether and, optionally, at least one (meth)acrylic ester, with at least one polyisocyanate in the presence of a catalyst other than tin.

6. The composition of claim 1 wherein the vinyl functional urethane is prepared by reacting at least one hydroxyl-containing polyester and least one polyisocyanate in the absence of a catalyst.

7. The composition of claim 1 wherein the vinyl functional urethane is prepared in the presence of a catalyst selected from the salts or complexes of an anion selected from the group consisting of bismuth, zinc, hafnium, zirconium, copper, iron, chromium, aluminum, cerium, titanium, manganese, nickel, cobalt, zinc, and potassium, or an amine catalyst.

8. The composition of claim 1 adapted for use as a builder gel wherein the initial viscosity is 250 - 2500 poise.

9. The composition of claim 1 adapted for use as a topcoat nail gel wherein the initial viscosity is 5-25 poise.

10. The composition of claim 1 adapted for use as a pigment containing polish gel wherein the initial viscosity is 10-40 poise.

11. The composition of claim 1 **characterized in that** it does not lose more than 5% of its viscosity within one year of storage.

12. The composition of claim 1 further comprising a solvent selected from the group consisting of butyl acetate, ethyl acetate, isopropanol, toluene, methyl ethyl ketone, acetone, xylene, propyl acetate, butanol, diacetone alcohol, propylene glycol, and butyl carbitol.

13. The composition of claim 1 comprising 5-80 wt. % (a), 20-80 wt. % (b), and 0.1 to 10 wt. % (c).

14. A method of coating a human finger nail or toe nail comprising applying a composition according to claim 1 to the finger or toe nail and curing in the presence of UV radiation.
